# EUROPEAN PATENT SPECIFICATION

(11) **EP 2 967 063 B1**
(45) Date of publication and mention of the grant of the patent: **18.10.2017**
(21) Application number: 14710530.8
(22) Date of filing: 12.03.2014
(51) Int. Cl.: A01N 43/56, A01P 21/00, A01P 3/00

(54) **LAWN GROWTH-PROMOTING AGENT AND METHOD OF USING SAME**
RASENWACHSTUMSFÖRDERNDER WIRKSTOFF UND VERFAHREN ZUR VERWENDUNG DAVON
AGENT FAVORISANT LA CROISSANCE DE GAZON ET SON PROCÉDÉ D'UTILISATION

(30) Priority: 13.03.2013 WO PCT/JP2013/049975
(43) Date of publication of application: 20.01.2016
(73) Proprietor: Bayer CropScience AG, 40789 Monheim (DE)
(72) Inventor: OHTAKE, Hirohisa, Sagamihara-shi Kanagawa 252-0184 (JP); NAKAMURA, Shin, Saitama Saitama 330-0073 (JP); YAMAMOTO, Hideki, Oyama-shi Tochigi 323-0829 (JP)
(74) Representative: BIP Patents
(86) International application number: PCT/EP2014/054858
(87) International publication number: WO 2014/140111

(56) References cited:
- WO-A2-2009/098218
- US-A1- 2010 216 636
- US-A1- 2010 298 139
- Anonymous: "Benefits of Plant Growth Regulators and What They Can Do For You", , 10 May 2010 (2010-05-10), XP055304712, Retrieved from the Internet: URL:https://web.archive.org/web/2010051008 3404/http://www.lawn-care-academy.com/plan t-growth-regulators.html [retrieved on 2016-09-22]

## Description

### TECHNICAL FIELD

The present invention relates to the use of penflufen to promote lawn growth.

### BACKGROUND ART

The use of growth-promoting agents is as important a part of maintaining golf courses, sports facilities, public parks, road embankments, and other lawned areas as fertilization, weed prevention and removal, pest control, watering, resoiling, mowing, aeration, slicing, thatching, and so on.

Conventionally, chlorella extract (trade name: Green Edge), *shiitake* extract (trade name: Lentimin), and mixed herbal extracts (trade name: Amgreen), have been used, but there is a demand for an even more effective lawn growth-promoting agent.

In particular, one important characteristic demanded of lawn growth-promoting agents is the ability to promote the growth of the roots of the lawn, improving the water absorption, nutrient absorption, and root spread of the lawn, while suppressing above-ground lawn growth. If lawn propagation is effected through vegetative propagation (sodding), i.e., through runner elongation (internode elongation), promoting the growth of lawn at the areas of new elongation is vital.

WO 2005/018324 discloses a method of promoting plant growth using a particular carboxamide. WO 2008/148482 and WO 2008/148476 discuss in general the supportive effects of penflufen upon crop growth. WO 2011/003533 has a specific disclosure regarding the promotion of soybean germination and seedling growth using penflufen. However, patent document 1 does not specifically disclose or even suggest the growth promotion effects of penflufen in the context of lawns. US 2010/298139 and US 2010/216636 report on the use of penflufen as a fungicide on lawns but neither document suggests its growth promotion effects.

### PRIOR ART REFERENCES

### PATENT REFERENCES

Patent Reference 1: Pamphlet of International Publication WO 2005/018324
Patent Reference 2: Pamphlet of International Publication WO 2008/148482
Patent Reference 3: Pamphlet of International Publication WO 2008/148476
Patent Reference 4: Pamphlet of International Publication WO 2011/003533
Patent Reference 5: Pamphlet of US publication US 2010/298139
Patent Reference 6: Pamphlet of US publication US 2010/216636

### SUMMARY OF THE INVENTION

### PROBLEMS TO BE SOLVED BY THE INVENTION

An object of the present invention is to promote the growth of lawn roots in order to improve root spread, and to provide a growth-promoting agent enabling lawn growth promotion in sodded areas in cases in which lawn propagation is via vegetative propagation (sodding), i.e., through runner elongation (internode elongation), as well as a method of using the same.

### MEANS USED TO SOLVE THE ABOVE-MENTIONED PROBLEMS

Therefore, the present invention provides a method of using a compound represented by formula (I): 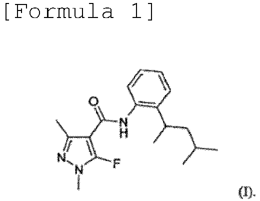 (chemical name: N-[2-(1, 3-dimethyl butyl) phenyl]-5-fluoro-1, 3-dimethyl-1H-pyrazol-4-carboxamide; generic name: penflufen) or an agriculturally acceptable salt thereof in order to promote lawn growth.

### BEST MODE FOR CARRYING OUT THE INVENTION

Penflufen is a known compound, and can be produced according to the method disclosed in WO 2006/092291.

Penflufen is also known as a pest-preventing agent for plants, and is extremely effective against brown patch (*Rhizoctonia solani*) (WO 2003/010149).

In the present invention, penflufen unexpectedly exhibits lawn growth-promoting effects, especially root growth-promoting effects promoting growth in newly sodded areas.

Thus, penflufen is extremely advantageous and effective both as a plant pest control agent and a lawn growth-promoting agent.

Penflufen can be converted to typical formulations such as solutions, emulsions, water dispersible agents, aqueous suspensions, oily suspensions, pulverized agents, powders, pastes, soluble powders, soluble granules, spreadable granules, suspoemulsions, natural compounds impregnated with active compounds, synthetic fertilizers impregnated with active compounds, and microcapsulized preparations in polymer materials.

These formulations can be combined with expanders for the active compounds (i.e., fluid solvents and/or solid carriers) using a surfactant (i.e., an emulsifying agent and/or dispersing agent and/or foaming agent) as necessary. Such formulations can be prepared in a suitable facility, or prepared prior to or during use.

Substances suitable for use as adjuvants are substances suitable for imparting the composition itself and/or preparations derived therefrom (for example, spray solutions, seed powders, etc.) with specific properties such as specific technical properties and/or specific biological properties. Typical suitable adjuvants include expanders, solvents, and carriers.

Examples of suitable expanders include water, and polar or non-polar organic chemical liquids such as ones selected from the following: aromatic and non-aromatic hydrocarbons (for example, paraffins, alkylbenzenes, alkylnaphthenes, and chlorobenzenes), alcohols and polyols (which may be substituted, etherified, or esterified as appropriate), ketones (for example, acetone and cyclohexanone), esters (including fatty acid esters and oil esters), (poly)ethers, substituted or unsubstituted amines, amides, and lactams (for example, N-alkyl pyrrolidones); and lactones, sulfones, and sulfoxides (for example, dimethyl sulfoxide).

If water is used as an expander, an organic solvent, for example, may be used as an auxiliary solvent. Essentially suitable liquid solvents include aromatic compounds such as xylenes, toluenes, and alkylnaphthalenes; chlorinated aromatic compounds and chlorinated aliphatic hydrocarbons such as chlorobenzenes, chloroethylenes, and methylene chlorides; aliphatic hydrocarbons such as cyclohexanes and paraffins, including petroleum fractions, mineral oils, and vegetable oils; alcohols such as butanols, glycols, and ethers and esters thereof; ketones such as acetone, methyl ethyl ketone, methyl isobutyl ketone, and cyclohexanone; and strongly polar solvents such as dimethyl sulfoxide; as well as water.

According to the present invention, a carrier is a natural or synthetic organic or inorganic substance, capable of being a solid or a liquid, that is mixed with or bonded to the active compound in order to improve ease of application, especially to plants or parts of plants. Such solid and liquid carriers should generally be inert and suitable for use on lawns.

Examples of suitable solid carriers include ammonium salts; pulverized natural minerals such as kaolin, clay, talc, chalk, quartz, attapulgite, montmorillonite, and diatomaceous earth; pulverized synthetic substances such as highly dispersed silica, alumina, and silicate. Examples of solid carriers suitable for granules include pulverized and segregated natural rock, such as calcite, marble, pumice, sepiolite, and dolomite; synthetic granules comprising organic or inorganic rough powders; and granules of organic materials such as paper, sawdust, coconut shell, corn cobs, and tobacco leafstalks. Suitable emulsifying agents and/or foaming agents include nonionic and anionic emulsifying agents, such as polyoxyethylene fatty acid esters, polyoxyethylene fatty acid alcohol ethers such as alkylaryl polyglycol ethers, alkyl sulfonates, alkyl sulfates, aryl sulfonates; and hydrolyzed proteins. Suitable dispersing agents include nonionic and/or ionic substances such as alcohol-POE and/or POP ethers, acid and/or POP-POE esters, alkyl aryl and/or POP-POE ethers, fatty and/or POP-POE adducts, POE- and/or POP- polyol derivatives, POE- and/or POP sorbitans and monosaccharide adducts, alkyl sulfates or aryl sulfates, alkyl sulfonates or aryl sulfonates, and alkyl phosphates or aryl phosphates, and corresponding POP ether adducts thereof. Further examples include suitable oligomers or polymers, such as vinyl monomer derivatives, acrylic acid derivatives, and derivatives of EO and/or PO alone or combined with, for example, (poly)alcohols or (poly)amines. Additionally, lignin and sulfonic acid derivatives thereof, unmodified cellulose and modified cellulose, aromatic and/or aliphatic sulfonic acid and formaldehyde adducts thereof, and the like can be used.

In the abovementioned formulations, a tackifier can be used, such as carboxymethyl cellulose natural and synthetic polymers in the form of a powder, granules, or latex, such as gum arabic, a polyvinyl alcohol, and polyvinyl acetate; natural phosphatides such as cephalin and lecithin; and synthetic phosphatides.

Colorants, including inorganic pigments such as iron oxide, titanium oxide, and prussian blue; organic dyes such as alizarin dyes, azo dyes, and metallic phthalocyanine dyes; and micronutrients such as iron salts, manganese salts, boron salts, copper salts, cobalt salts, molybdenum salts, and zinc salts, can be used.

Other possible additives include aromatic compounds; mineral oils or vegetable oils that may be modified as necessary; waxes, nutrients (including micronutrients) such as iron salts, manganese salts, boron salts, copper salts, cobalt salts, molybdenum salts, and zinc salts; and the like.

Stabilizers (for example, low-temperature stabilizers), preservatives, antioxidants, photostabilizers, and other agents that improve chemical and/or physical stability may also be included.

The abovementioned formulations comprise 0.01-98 wt% penflufen, preferably 0.5-90 wt% penflufen.

In an embodiment of the present invention, penflufen may be used alone, or may be comprised in a formulation, encompassing mixtures of secondary ingredients such as one or more type of suitable germicide, bactericide, acaricide, nematicide, insecticide, microbicide, fertilizer, attractant, sterilant, synergist, safener, information chemical substance, and/or plant growth adjuster. Combining the penflufen of the present invention and secondary ingredients yields synergistic effects. In other words, the activity of the mixture is greater than the activity that would be expected on the basis of the activity of the individual ingredients. Generally, said combination can be used in a premix, tank mix, or pre-prepared mixture.

Broadly speaking, the various additional secondary ingredients can preferably be mixed with the penflufen of the present invention at a ratio of 100:1-1:100, especially preferably 5:1-1:5.

In one embodiment of the present invention, penflufen is combined with one or more active ingredients selected from the group consisting of germicides, bactericides, acaricides, nematicides, insecticides, microbicides, fertilizers, attractants, sterilants, synergists, safeners, information chemical substances, and plant growth adjusters, and used to promote lawn growth.

Non-limiting examples of secondary ingredients include the following.

Insecticides/acaricides/nematicides: The active compounds identified by their generic names within the present specification are known, and are listed, for example, in agricultural chemical handbooks ("The Pesticide Manual" 14th Ed., British Crop Protection Council 2006), or can be found online (for example, http://www.alanwood.net/pesticides).
(1) Acetylcholinesterase (AChE) inhibitors, including carbamate-based inhibitors such as alanycarb, aldicarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, mesomil, metolcarb, oxamyl, pirimicarb, propoxur, thiodicarb, thiofanox, triazamate, trimetacarb, XMC, and xylylcarb; and
   organic phosphate ester-based inhibitors, such as acephate, azamethiphos, azinphos(-methyl, -ethyl), cadusafos, chlorethoxyfos, chlorphenvinphos, chlormephos, chlorpyrifos (-methyl), coumaphos, cyanohos, dimethone-s-methyl, diazinon, dichlorvos/DDVP, dicrotophos, dimethoate, dimethylvinphos, disulfoton, EPN, ethion, ethoprophos, famphur, fenamiphos, fenitrothion, fenthion, fosthiazate, heptenophos, isophenphos, isopropyl 0-(methoxyamine thiophosphoryl) salicylate, isoxathion, malathion, mecalpam, methamidophos, methidathion, mevinphos, monocrotophos, naled, omethoate, oxydemeton-methyl, parathion (-methyl), phenthoate, phorate, phosalone, phosmet, phosphamidon, phoxim, pyrimiphos (-methyl), profenofos, propetamphos, prothiophos, pyraclofos, pyridafenthion, quinalphos, sulfotep, tebupirimphos, temephos, terbufos, tetrachlorfenvinphos, thiometone, triazophos, trichlorfon, and vamidothion;
(2) GABA-gated chloride channel antagonists, including organic chlorides such as chlordane and endosulfan (alpha-); and fiproles (phenyl pyrazoles) such as ethiprole, fipronil, pyrafluprole, and pyriprole;
(3) sodium channel modulators / voltage-dependent sodium channel blockers, including:
   pyrethroids, such as acrinathrin, allethrin (d-cis-trans, d-trans), bifenthrin, bioallethrin, bioallethrin-s-cyclopentenyl, bioresmethrin, cycloprothrin, cyfluthrin (beta), cyhalothrin (gamma,lambda), cypermethrin, (alpha, beta, theta, zeta), cyphenothrin [(1R)-trans-isomer], deltamethrin, dimefluthrin, empenthrin [(EZ)-(1R)-isomer], esfenvalerate, etofenprox, fenpropathrin, fenvalerate, fenvalerate, flumethrin, fluvalinate (tau-), halfenprox, imiprothrin, metofluthrin, permethrin, phenothrin [(1R)-trans-isomer)], prallethrin, profluthrin, pyrethrin (pyrethrum), resmethrin, RU 15525, silafluofen, tefluthrin, tetramethrin [(1R)-isomer], tralomethrin, transfluthrin, ZXI 8901, and DDT; and methoxychlor;
(4) nicotinergic acetylcholine receptor agonists, including:
   neonicotinoids such as acetamiprid, clothianidin, dinotefuran, imidacloprid, nitenpyram, thiacloprid, and thiamethoxam; or nicotine;
(5) allosteric acetylcholine receptor modulators (agonists), including:
   spinosyns scuh as spinetoram and spinosad;
(6) chloride channel activators, including avermectin-based and milbemycin-based, such as:
   abamectin, emamectin benzoate, lepimectin, and milbemectin;
(7) juvenile hormone analogues, including:
   hydroprene, quinoprene, and methoprene; fenoxycarb; and pyriproxyfen;
(8) active compounds for which the mechanism of action is not known or non-specific active compounds, including:
   fumigants such as methyl borate and other halogenated alkyls; and
   chloropicrin; sulfuryl fluoride; borax; antimony potassium tartrate;
(9) selective antifeedants, including:
   pymetrozine and flonicamid;
(10) mite growth inhibitors, including:
   clofentezine, diflovidazin, hexythiazox, and ethoxazole;
(11) microbial insect digestive tract membrane disruptors, including;
   *Bacillus thuringiensis subspecies israelensis, Bacillus sphaericus, Bacillus thuringensis subspecies aizawai, Bacillus thuringiensis subspecies kurstaki, and Bacillus thuringiensis subspecies tenebrionis;* and BT plant proteins such as Cry1Ab, Cry1Ac, Cry1Fa, Cry2Ab, mCry3A, Cry3Ab, Cry3Bb, and Cry34/35Ab1;
(12) oxidative phosphorylation inhibitors and ATP distruptors, including:
   diafenthiuron; and organotin compounds such as azocyclotin, cyhexatin, and fenbutatin oxide; and
   propargite and tetradifon;
(13) oxidative phosphorylation decouplers that function by blocking H+ proton gradients, including;
   chlorfenapyr and DNOC;
(14) nicotinergic acetylcholine receptor blockers, including:
   bensultap, cartap (hydrochloride), thiocyclam, thiosultap (sodium);
(15) chitin biosynthesis inhibitors (type 0), including:
   benzoyl urea-based inhibitors, such as bistrifluron, chlorfluazuron, diflubenzuron, flucycloxuron, flefenoxuron, hexaflumuron, lufenuron, novaluron, noviflumuron, teflubenzuron, and triflumuron;
(16) chitin biosynthesis inhibitors (type 1), including:
   buprofezin;
(17) moulting disruptors, including:
   cyromazine;
(18) ecdysone agonists/disruptors, including:
   diacylhydrazine-based, such as chromafenozide, halofenozide, methoxyfenozide, and tebufenozide;
(19) octopamine agonists, including:
   amitraz;
(20) Electron transfer complex III inhibitors, including:
   hydramethylnon; acequinocyl; fluacrypyrim; and flometoquin;
(21) Electron transfer complex I inhibitors, including:
   those belonging to the METI acaricides, such as fenazaquin, fenpyroximate, pyrimidifen, pyridaben, tebufenpyrad, and tolefenpyrad; and rotenone (Derris);
(22) voltage-dependent sodium channel blockers, including:
   indoxacarb and metaflumizone;
(23) acetyl CoA carboxylase inhibitors, including;
   tetronic acid derivatives, such as spirodiclofen and spiromesifen; or tetramic acid derivatives such as spirotetramate;
(24) Electron transfer complex IV inhibitors, including:
   phosphine-based, such as aluminum phosphate, calcium phosphate, phosphine, and zinc phosphate; and cyanates;
(25) Electron transfer complex II inhibitors, including:
   cyenopyrafen; cyflumetofen; pyflubumide;
(28) ryanodine receptor effectors, including;
   diamide-based, such as flubendiamide, chlorantraniliprole (Rynaxypyr), and cyantraniliprole (Cyazypyr); and 3-bromo-N- {2-bromo-4-chloro-6-[(1-cyclopropylethyl)carbamoyl]phenyl}-1-(3-chloropyridine-2-yl)-1H-pyrazol-5-carboxamide (known from WO 2005/077934), and 2-[3,5-dibromo-2-({[3-bromo-1-(3-chloropyridine-2-yl)-1H-pyrazol-5-yl]carbonyl}amino)benzoyl]-1, 2-dimethylhydrazine methyl carboxylate (known from WO 2007/043677). IKI-3106 liquid 50 Active compounds for which (New Agrochemical Practical Application Test Results) the mechanism of action is unknown, including azadirachtin, amidoflumet, benzoximate, bifenazate, quinomethionate, cryolite, dicofol, fluensulfone (5-chloro-2-[(3,4,4-trifluorobuta-3-en-1-yl)sulphonyl]-1,3-thiazol), flufenerim, fluopyram, pyridalyl, and pyrifluquinazon; and products based on Bacillus firmus (1-1582, Bioneem, Votivo); and the following known active compounds:
      4-{[(6-bromopyrida-3-yl)methyl](2-fluoroethyl)amino)furan2-(5H)-on (known from WO 2007/115644), 4-{[(6-fluoropyrida-3-yl)methyl](2,2-difluoro ethyl)amino}furan-2(5H)-on (known from WO 2007/115644), 4-{[(2-chloro-1,3-thiazol-5-yl)methyl](2-fluoroethyl)amino} furan-2(5H)-on (known from WO 2007/115644), 4-{[(6-chloropyrida-3-yl)methyl](2-fluoroethyl)amino}furan-2(5H)-on (known from WO 2007/115644), 4-{[(6-chloropyrida-3-yl)methyl](2,2-difluoroethyl)amino}furan-2(5H)-on (known from WO 2007/115644), 4-{[(6-chloro-5-fluoropyrida-3-yl)methyl](methyl)amino}furan-2(5H)-on (known from WO 2007/115643), 4-{[(5,6-dichloropyrida-3-yl)methyl](2-fluoroethyl)amino}furan-2(5H)-on (known from WO 2007/115646), 4-{[(6-chloro-5-fluoropyrida-3-yl)methyl](cyclopropyl)amino} furan-2(5H)-on (known from WO 2007/115643), 4-{[(6-chloropyrida-3-yl)methyl](cyclopropyl)amino}furan-2(5H)-on (known from EP-A-0539588), 4-{[(6-chloropyrida-3-yl)methyl](methyl)amino}furan-2(5H)-on (known from EP-A-0539588), [(6-chloropyridine-3-yl)methyl](methyl)oxide-λ⁴-sulfaniliden cyanamide (known from WO 2007/149134), [1-(6-chloropyridine-3-yl)ethyl](methyl)oxide-λ⁴-sulfaniliden cyanamide (known from WO 2007/149134), and its diastereomers (A) and (B). 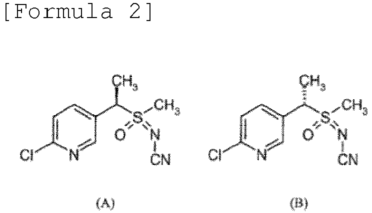
(likewise known from WO 2007/149134), [(6-trifluoromethyl pyridine-3-yl)methyl](methyl)oxide-λ⁴-sulfaniliden cyanamide (known from WO 2007/095229), sulfoxaflor (likewise known from WO 2007/149134), 11-(4-chloro-2,6-dimethyl phenyl)-12 hydroxy-1,4-dioxa-9-azadispiro[4.2.4.2]tetradeca-11-en-10-on (known from WO 2006/089633), 3-(4'-fluoro-2,4-dimethyl biphenyl-3-yl)-4-hydroxy 8-oxa-1-azaspiro[4.5]deca-3-en-2-on (known from WO 2008/067911), 1-[2-fluoro-4-methyl-5-[(2,2,2-trifluoroethyl)sulfinyl]phenyl]-3-(trifluoro methyl)-1H-1,2,4-triazole-5-amine (known from WO 2006/043635), [(3S,4aR,12R,12aS,12bS)-3-[(cyclopropylcarbonyl)oxy]-6,12-dihydroxy-4,12b-dimethyl-11-oxy-9-(pyridine-3-yl)-1,3,4,4a,5,6, 6a,12,12a,12b-decahydro-2H,11H-beno[f]pyrano[4,3-b]chromene-4-yl]methyl cyclopropane carboxylate (known from WO 2006/129714), 2-cyano-3-(difluoromethoxy)-N,N-dimethyl benzene sulfonamide (known from WO 2006/056433), 2-cyano-3-(difluoromethoxy)-N-methyl benzene sulfonamide (known from WO 2006/100288), 2-cyano-3-(difluoromethoxy)-N-ethyl benzene sulfonamide (known from WO 2005/035486), 4-(difluoromethoxy)-N-ethyl-N-methyl-1,2-benzothiazol-3-amine 1,1-dioxide (known from WO 2007/057407), and N-[1-(2,3-dimethylphenyl)-2-(3,5-dimethylphenyl)ethyl]-4,5-dihydro-1,3-thiazol-2-amine (known from WO 2008/104503). Especially preferable secondary ingredients include: chlorantraniliprole, thiodicarb, cyfluthrin, imidacloprid, thiacloprid, cyfluthrin, silafluofen, acetamiprid, thiamethoxam, flubendiamide, permethrin, methoxyfenozide, etofenprox, teflubenzuron, diazinon, tralomethrin, BT, indoxacarb, clothianidin, pyrimiphos methyl, bensultap, and the like, but the present invention is not limited to these.

### Germicides

(1) Ergosterol biosynthesis inhibitors, including: aldimorph, azaconazole, bitertanol, bromuconazole, cyproconazole, diclobutrazole, difenoconazole, diniconazole, diniconazole-M, dodemorph, dodemorph acetate, epoxyconazole, etaconazole, fenarimol, fenbuconazole, fenhexamid, fenpropidin, fenpropymorph, fluquinconazole, flurprimidol, flusilazole, flutriafol, fluconazole, fluconazole-cis, hexaconazole, imazalil, imazalil sulfate, imibenconazole, ipconazole, metconazole, myclobutanil, naftifine, nuarimol, oxpoconazole, paclobutrazol, pefurazoate, penconazole, piperalin, prochloraz, propiconazole, prothioconazole, pyributycarb, pyrifenox, quinconazole, simeconazole, spiroxamine, tebuconazole, terpinafin, tetraconazole, triadimefon, triadimenol, tridemorph, triflumizole, triphorin, triticonazole, uniconazole, uniconazole-p, biniconazole, polyconazole, 1-(4-chlorophenyl)-2-(1H-1,2,4-triazole-1-yl)cycloheptanol, 1-(2,2-dimethyl-2,3-dihydro-1H-indene-1-yl)-1H-imidazole-5-methylcarboxylate, N'-{5-(difluoromethyl)-2-methyl-4-[3-(trimethylsilyl)propoxy]phenyl}-N-ethyl-N-methylimidoformamide, N-ethyl-N-methyl-N'-{2-methyl-5-(trifluoromethyl)-4-[3-(trimethylsilyl)propoxy]phenyl)imidoformamide, and O-[1-(4-methoxyphenoxy)-3,3-dimethylbutane-2-yl]-1H-imidazole-1-carbothioate.
(2) Respiratory inhibitors (respiratory chain inhibitors), including: bixafen, boscalid, carboxin, dliflumetorim, fenfuram, fluopyram, flutranil, fluxapyroxad, furametpyr, furmecyclox, antiepimeric racemes 1RS, 4SR, 9RS and isopyrazam mixtures of antiepimeric racemes 1RS, 4SR, 9SR, isopyrazam (antiepimeric racemes), isopyrazam (antiepimeric enantiomers 1R, 4S, 9S), isopyrazam (antiepimeric enantiomers 1S, 4R, 9R), isopyrazam (antiepimeric enantiomers 1RS, 4SR, 9RS), isopyrazam (antiepimeric enantiomers 1R,4S,9R), isopyrazam (antiepimeric enantiomers 1S, 4R, 9S), mepronil, oxycarboxin, penflufen, penthiopyrad, sedaxane, thifluzamide, 1-methyl-N-[2-(1,1,2,2-tetrafluoroethoxy)phenyl]-3-(trifluoromethyl)-1H-pyrazol-4-carboxamide, 3-(difluoromethyl)-1-methyl-N-[2-(1,1,2,2-tetrafluoroethoxy)phenyl]-1H-pyrazol-4-carboxamide,3-(difluoromethyl)-N-[4-fluoro-2-(1,1,2,3,3,3-hexafluoropropoxy)phenyl]-1-methyl-1H-pyrazol-4-carboxamide, and N-[1-(2,4-dichlorophenyl)-1-methoxypropane-2-yl]-3-(difluoromethyl)-1-methyl-1H-pyrazol-4-carboxamide.
(3) Respiratory inhibitors (respiratory chain inhibitors) affecting respiratory chain complex III, including ametoctradin, amisulbrom, azoxystrobin, cyazofamid, dimethoxystrbin, enestroburin, famoxadone, fenamidone, fluoxastrobin, kresoximmethyl, metominostrobin, orysastrobin, picoxystrobin, pyraclostrobin, pyrametostrobin, pyraoxystrobin, pyribencarb, rifloxystrobin, (2E)-2-(2-{[6-(3-chloro-2-methylphenoxy)-5-fluoropyrimidine-4-yl]oxy)phenyl)-2-(methoxyimino)-N-methylethanamide, (2E)-2-(methoxyimino)-N-methyl-2-(2-{[({(1E)-1-[3-(trifluoromethyl)phenyl]ethylidene}-amino)oxy]methyl}phenyl)ethanamide, (2E)-2-(methoxyimino)-N-methyl-2-{2-[(E)-({1-[3-(trifluoromethyl)phenyl]ethoxy}imino)-methyl]phenyl}ethanmide, (2E)-2-{2-[({[(1E)-1-(3-{[(E)-1-fluoro-2-phenylethenyl]oxy}phenyl)ethylidene]amino}oxy)-methyl]phenyl}-2-(methoxyimino)-N-methylethanamide, (2E)-2-{2-[({[(2E,3E)-4-(2,6-dichlorophenyl)buto-3-en-2-ylidene]-amino)oxy)methyl]phenyl}-2-(methoxyimino)-N-methylethanamide, 2-chloro-N-(1,1,3-trimethyl-2,3-dihydro-1H-indene-4-yl)pyridine-3-carboxamide,5-methoxy-2-methyl-4-(2-{[({(1E)-1-[3-(trifluoromethyl)phenyl]ethylidene)amino)oxy]methyl}-phenyl)-2,4-dihydro-3H-1,2,4-triazole-3-on,methyl(2E)-2-{2-[({cyclopropyl[(4-methoxyphenyl)imino]methyl}sulfanil)-methyl]phenyl}-3-methoxyprop-2-enate,N-(3-ethyl-3,5,5-trimethylcyclohexyl)-3-(formylamino)-2-hydroxybenzamide,2-{2-[(2,5-dimethylphenoxy)methyl]phenyl}-2-methoxy-N-methylacetamide,and(2R)-2-{2-[(2,5-dimethylphenoxy)methyl]-phenyl}-2-methoxy-N-methylacetamide.
(4) Mitosis and cell division inhibitors, including: benomyl, carbendazim, chlorfenazole, diethofencarb, ethaboxam, fluopicolide, fuberidazole, pencycuron, thiabendazole, thiophanate-methyl, thiophanate, zoxamide, 5-chloro-7-(4-methylpiperidine-1-yl)-6-(2,4,6-trifluorophenyl)[1,2,4]triazolo[1,5-a]pyrimidine, and 3-chloro-5-(6-chloropyridine-3-yl)-6-methyl-4-(2,4,6-trifluorophenyl)pyridazine.
(5) Compounds having multisite activity, including: Bordeaux mix, captafol, captan, chlorothalonil; copper hydroxide, copper napthenate, copper oxide, basic copper chloride, copper sulfate, and other copper preparations; dichlofluanid, dithianon, dodine, dodine free base, ferba, fluor-folpet, forpet, guazatine, guazatine acetate, iminoctadine, iminoctadine albesilate, iminoctadine triacetate, mancopper, mancozeb, maneb, metiram, metiram-zinc, oxine-copper, propamidine, propineb; sulfur and sulfur agents such as calcium polysulfide, thiuram, tolylfluanid, zineb, and ziram.
(6) Resistant derivatives, including acibenzolar-s-methyl, isotianil, probenazole, and tiadinil.
(7) Amino acid and protein biosynthesis inhibitors, including andoprim, blasticidin S, cyprodinil, kasugamycin, kasugamycin hydrochloride hydrate, mepanipyrim, and pyrimethanil.
(8) ATP production inhibitors, including fentin acetate, fentin chloride, fentin hydroxide, and silthiofan.
(9) Cell wall synthesis inhibitors, including benthiavalicarb, dimethomorph, flumorph, iprovalicarb, mandipropamid, polyoxins, polyoxorim, validamycin A, and valifenalate.
(10) Lipid and membrane synthesis inhibitors, including biphenyl, chloroneb, dicloran, edifenphos, etridiazole, iodocarb, iprobenfos, isoprothiolane, propamocarb, propamocarb hydrochloride, prothiocarb, pyrazophos, quintozene, tecnazene, and tolclofos-methyl.
(11) Melanin biosynthesis inhibitors, including carpropamid, diclocymet, fenoxanil, phthalide, pyroquilone, and tricyclazole.
(12) Nucleic acid synthesis inhibitors, including benalaxyl, benalaxyl M (kiralaxyl), bupirimate, clozylacon, dimethirimol, ethirimol, furalaxyl, hymexazol, metalaxyl, metalaxyl M (mefenoxam), ofurace, oxadixyl, and oxolinic acid.
(13) Signal transduction inhibitors, including chlozolinate, fenpiclonil, fludioxonil, iprodione, procymidone, quinoxyfen, and vinclozolin.
(14) Uncoupling agents, including binapacryl, dinocap, ferimzone, fluazinam, and meptyldinocap.
(15) Other compounds, such as benthiazol, bethoxazin, capsimycin, carvone, quinomethinoate, chlazafenon, cufraneb, cyflufenamid, cymoxanil, cyprosulfamide, dazomet, debacarb, dichlorophen, diclomezine, difenzoquat methyl sulfate, diphenylamine, ecomat, fenpyrazamine, flumetover, fluoromide, flusulfamide, flutianil, fosetyl aluminum, fosetyl calcium, fosetyl sodium, hexachlorobenzene, irumamycin, methasulfocarb, methyl isothiocyanate, metrafenone, mildiomycin, natamycin, diethyl dithiocarbamate, nitrothal isopropyl, octhilinone, oxamocarb, oxyfenthiin, pentachlorophenol and salts thereof, phenothrin, phosphoric acid and salts thereof, propamocarb fosetylate, propanosine sodium, proquinazid, pyrrolnitrin, tebufloquin, tecloftalam, tolnifanide, triazoxide, trichlamide, zarilamid, 1-(4-{4-[(5R)-5-(2,6-difluorophenyl)-4,5-dihydro-1,2-oxazole-3-yl]-1,3-thiazol-2-yl}piperidine1-yl)-2-[5-methyl-3-(trifluoromethyl)-1H-pyrazol-1-yl]ethanone, 1-(4-{4-[(5S)-5-(2,6-difluorophenyl)-4,5-dihydro-1,2-oxazole-3-yl]-1,3-thiazol-2-yl)piperidine-1-yl)-2-[5-methyl-3-(trifluoromethyl)-1H-pyrazol-1-yl]ethanone,1-(4-{4-[5-(2,6-difluorophenyl)-4,5-dihydro-1,2-oxazole3-yl]-1,3-thiazol-2-yl}piperidine-1-yl)-2-[5-methyl-3-(trifluoromethyl)-1H-pyrazol-1-yl]ethanone, 1H-imidazole-1-carboxylicacid1-(4-methoxyphenoxy)-3,3-dimethylbutane-2-yl, 2,3,5,6-tetrachloro-4-(methylsulfonyl)pyridine, 2,3-dibutyl-6-chlorothieno[2,3-d]pyrimidine-4(3H)-one, 2-[5-methyl-3-(trifluoromethyl)-1H-pyrazol-1-yl]-1-(4-{4-[(5R)-5-phenyl-4,5-dihydro-1,2-oxazole-3-yl]-1,3-thiazol-2-yl)piperidine-1-yl)ethanone, 2-[5-methyl-3-(trifluoromethyl)-1H-pyrazol-1-yl]-1-(4-{4-[(5S)-5-phenyl-4,5-dihydro-1,2-oxazole-3-yl]-1,3-thiazol-2-yl}piperidine-1-yl)ethanone, 2-[5-methyl-3-(trifluoromethyl)-1H-pyrazol-1-yl]-1-{4-[4-(5-phenyl-4,5-dihydro-1,2-oxazole3-yl)-1,3-thiazol-2-yl]piperidine-1-yl}ethanone, 2-butoxy-6-iodine-3-propyl-4H-chromene-4-one, 2-chloro-5-[2-chloro-1-(2,6-difluoro-4-methoxyphenyl)-4-methyl-1H-imidazole-5-yl]pyridine,2-phenyl phenol and salts thereof, 3,4,5-trichloropyridine-2,6-dicarbonitrile 3-[5-(4-chlorophenyl)-2,3-dimethyl-1,2-oxazolidine-3-yl]pyridine, 3-chloro-5-(4-chlorophenyl)-4-(2,6-difluorophenyl)-6-methylpyridazine,4-(4-chlorophenyl)-5-(2,6-difluorophenyl)-3,6-dimethylpyridazine, 5-amino-1,3,4-thiadiazole-2-thiol,5-chloro-N'-phenyl-N'-(prop-2-in-1-yl)thiophene-2-sulfanohydrazide, 5-methyl-6-octyl[1,2,4]triazolo[1,5-a]pyrimidine-7-amine, ethyl(2Z)-3-amino-2-cyano-3-phenylprop-2-enate, N-(4-chlorobenzyl)-3-[3-methoxy-4-(prop-2-in-1-yloxy)phenyl]propaneamide, N-[(4-chlorophenyl)(cyano)methyl]-3-[3-methoxy-4-(prop-2-in-1-yloxy)phenyl]propaneamide, N-[(5-bromo-3-chloropyridine-2-yl)methyl]-2,4-dichloropyridine-3-carboxamide, N-[1-(5-bromo-3-chloropyridine-2-yl)ethyl]-2,4-dichloropyridine-3-carboxamide, N-[1-(5-bromo-3-chloropyridine-2-yl)ethyl]-2-fluoro-4-iodinepyridine-3-carboxamide, N-{(E)-[(cyclopropylmethoxy)imino][6-(difluoromethoxy)-2,3-difluorophenyl]methyl}-2-phenylacetamide, N-{(Z)-[(cyclopropylmethoxy)imino][6-(difluoromethoxy)-2,3-difluorophenyl]methyl}-2-phenylacetamide, N-methyl-2-(1-{[5-methyl-3-(trifluoromethyl)-1H-pyrazol-1-yl]acetyl)piperidine-4-yl)-N-(1,2,3,4-tetrahydronaphthalene-1-yl)-1,3-thiazol-4-carboxamide, N-methyl-2-(1-{[5-methyl-3-(trifluoromethyl)-1H-pyrazol-1-yl]acetyl}piperidine-4-yl)-N-[(1R)-1,2,3,4-tetrahydronaphthalene-1-yl]-1,3-thiazol-4-carboxamide, N-methyl-2-(1-{[5-methyl-3-(trifluoromethyl)-1H-pyrazol-1-yl]acetyl}piperidine-4-yl)-N-[(1S)-1,2,3,4-tetrahydronaphthalene-1-yl]-1,3-thiazol-4-carboxamide, pentyl{6-[({[(1-methyl-1H-tetrazol-5-yl)(phenyl)methylidene]amino)oxy)methyl]pyridine-2-yl} carbamide, fenadine-1-carboxylic acid, quinoline-8-ole, and quinoline-8-ole sulfate (2:1).
(16) Other compounds, including 1-methyl-3-(trifluoromethyl)-N-[2'-(trifluoromethyl)biphenyl-2-yl]-1H-pyrazol-4-carboxamide, N-(4'-chlorobiphenyl-2-yl)-3-(difluoromethyl)-1-methyl-1H-pyrazol-4-carboxamide, N-(2',4'-dichlorobiphenyl-2-yl)-3-(difluoromethyl)-1-methyl-1H-pyrazol-4-carboxamide, 3-(difluoromethyl)-1-methyl-N-[4'-(trifluoromethyl)biphenyl-2-yl]-1H-pyrazol-4-carboxamide, N-(2',5'-difluorobiphenyl-2-yl)-1-methyl-3-(trifluoromethyl)-1H-pyrazol-4-carboxamide,3-(difluoromethyl)-1-methyl-N-[4'-(prop-1-in-1-yl)biphenyl-2-yl]-1H-pyrazol-4-carboxamide, 5-fluoro-1,3-dimethyl-N-[4'-(prop-1-in-1-yl)biphenyl-2-yl]-1H-pyrazol-4-carboxamide, 2-chloro-N-[4'-(prop-1-in-1-yl)biphenyl-2-yl]pyridine-3-carboxamide,3-(difluoromethyl)-N-[4'-(3,3-dimethylbut-1-in-1-yl)biphenyl-2-yl]-1-methyl-1H-pyrazol-4-carboxamide, N-[4'-(3,3-dimethylbut-1-in-1-yl)biphenyl-2-yl]-5-fluoro-1,3-dimethyl-1H-pyrazol-4-carboxamide, 3-(difluoromethyl)-N-(4'-ethynylbiphenyl-2-yl)-1-methyl-1H-pyrazol-4-carboxamide, N-(4'ethynylbiphenyl-2-yl)-5-fluoro-1,3-dimethyl-1H-pyrazol-4-carboxamide, 2-chloro-N-(4'-ethynylbiphenyl-2-yl)pyridine-3-carboxamide, 2-chloro-N-[4'-(3,3-dimethylbut-1-in-1-yl)biphenyl-2-yl]pyridine-3-carboxamide, 4-(difluoromethyl)-2 methyl-N-[4'-(trifluoro-methyl)biphenyl-2-yl]-1,3-thiazol-5-carboxamide, 5-fluoro N-[4'-(3-hydroxy-3-methylbut-1-in-1-yl)biphenyl-2-yl]-1,3-dimethyl-1H-pyrazol-4-carboxamide, 2-chloro-N-[4'-(3-hydroxy-3-methylbut-1-in-1-yl)biphenyl-2-yl]pyridine-3-carboxamide, 3-(difluoromethyl)-N-[4'-(3-methoxy-3-methylbut-1-in-1-yl)biphenyl-2-yl]-1-methyl-1H-pyrazol-4-carboxamide,5-fluoro-N-[4'-(3-methoxy-3-methylbut-1-in-1-yl)biphenyl-2-yl]-1,3-dimethyl-1H-pyrazol-4-carboxamide, 2-chloro-N-[4'-(3-methoxy-3-methylbut-1-in-1-yl)biphenyl-2-yl]pyridine-3-carboxamide, and (5-bromo-2-methoxy-4-methylpyridine-3-yl)(2,3,4-trimethoxy-6-methylphenyl)methanone N-[2-(4-{[3-(4-chlorophenyl)prop-2-in-1-yl]oxy}-3-methoxyphenyl)ethyl]-N2-(methylsulfonyl) valinamide. BAF-1207 flowable (New Agrochemical Practical Application Test Results, published by the Japan Plant Protection Association), NF-171 granular hydrate agent 20 (ibid.), GG226SC (ibid.), NNF-0721 flowable 20 (ibid.), NR-29 hydrate agent (ibid.).

Especially preferable secondary ingredients are thifluzamide, polyoxin-D zinc salt, tolclofos methyl, boscalid, captan, organic copper preparations, penthiopyrad, iminoctadine acetate, thiophanate methyl, isoprothiolane, fosetyl, flutranil, tebuconazole, polycarbamate, metalaxyl, metconazole, azoxystrobin, cyproconazole, hexaconazole, mepronil, pencycuron, chloroneb, procamocarb, Kresoxim methyl, difenoconazole, TPN, hydroxy isoxazole, iprodione, imibenconazole, metconazole, propiconazole, triadimefon, bitertanol, simeconazole, validamycin, propineb, amisulbrom, oxpoconazole fumarate, benomyl, manzeb, fludioxonil, myclobutanil, cyazofamid, furametpyr, trifloxistrobin, and the like, but the present invention is not limited thereto.

### Herbicide and plant modifiers

These are known active compounds that inhibit, for example, acetolactate synthase, acetyl-CoA carboxylase, cellulose synthase, enolpyruvylshikimate-3-phosphate synthase, glutamine synthase, p-hydroxy phenylpyruvate dioxygenase, phytoene desaturase, photosystem I, photosystem II, and protoporphyrinogen oxidase, and are disclosed, for example, in Weed Research 26 (1986) pp. 441-445, or "The Pesticide Manual," 14th edition, The British Crop Protection Council and the Royal Soc. of Chemistry, 2006, as well as the documents cited therein. Known herbicides and plant growth adjusters capable of being combined with the penflufen according to the present invention are, for example, the following active substances (compounds are listed according to their generic names according to International Organization for Standardization (ISO) standards, by chemical name, or by code number), and always include all usable forms thereof, such as acids, salts, esters, and isomers such as stereoisomers and optic isomers.

Acetochlor, acibenzolar, acibenzolar-S-methyl, acifluorfen, acifluorfen sodium, aclonifen, alachlor, allidochlor, alloxydym, alloxydym sodium, ametrine, amicarbazone, amidochlor, amidosulfone, aminocyclopyrachlor, aminopyralid, amitrole, ammonium sulfamate, ancymidol, anilofos, asulam, atrazine, azafenidin, azimsulfuron, aziprotryne, beflubutamid, benazolin, benazolin-ethyl, bencarbazone, benfluralin, benfuresate, bensulide, bensulfuron, bensulfuron methyl, bentazon, benzfendizone, benzobicyclon, benzofenap, benzofluor, benzoylprop, cyclopyrrolone, bifenox, bilanafos, bilanafos sodium, bispyribac, bispyribac sodium, bromacil, bromobutide, bromophenoxym, bromoxynil, bromuron, buminafos, busoxinone, butachlor, butafenacil, butamifos, butenachlor, butralin, butroxydim, butyrate, cafenstrole, carbetamide, carfentrazone, carfentrazone ethyl, clomethoxyfen, chloramben, chlorazifop, chlorazifop butyl, chlorbromuron, chlorbufam, chlorfenac, chlorfenac sodium, chlorfenprop, chlorflurenol, chlorflurenol methyl, chloridazon, chlorimuron, chlorimuron ethyl, chlormequat chloride, chloronitrofen, chlorophthalim, chlorthal dimethyl, chlorotoluron, chlorsulfuron, cinidon, cinidon ethyl, cinmethylin, cinosulfuron, clethodim, clodinafop, clodinafop propargyl, clofencet, clomazone, clomeprop, cloprop, clopyrarid, cloransulam, cloransulam methyl, cumyluron, cyanamide, cyanazine, cyclanilide, cycloate, cyclosulfamuron, cycloxydim, cycluron, cyhalofop, cyhalofop butyl, cyperquat, cyprazine, cyprazole, 2,4-D, 2,4-DB, daimuron/dymron, dalapon, daminozide, dazomet, n-decanol, desmedipham, desmetryne, detosyl pyrazolate (DTp), diallate, dicamba, dichlobenil, dichloroprop, dichloroprop-p, diclofop, diclofop methyl, diclofop-p-methyl, diclosulam, diethatyl, diethatyl ethyl, difenoxuron, difenzoquat, diflufenican, diflufenzopyr, diflufenzopyr sodium, dimefuron, dikegulac sodium, dimefuron, dimepiperate, dimethachlor, dimethametryn, dimethenamide, dimethenamide-p, dimethipin, dimetrasulfuron, dinitramine, dinoseb, dinoterb, diphenamid, dipropetryn, diquat, diquat dibromide, dithiopyr, diuron, triafamone, DNOC, eglinazine ethyl, endothal, EPTC, esprocarb, ethalfluralin, ethametsulfuron, ethametsulfuron methyl, ethephon, ethidimuron, ethiozin, ethofumesate, ethoxyfen, ethoxyfen ethyl, ethoxysulfuron, etobenzanide, F-5331, i.e., N-[2-chloro-4-fluoro-5-[4-(3-fluoropropyl)-4,5-dihydro-5-oxo-1H-tetrazole-1-yl]-phenyl]-ethane sulfonamide, F-7967, i.e., 3-[7-chloro-5-fluoro-2-(trifluoromethyl)-1H-benzimidazole-4-yl]-1-methyl-6-(trifluoromethyl)pyrimidine-2, 4(1H, 3H)-dione, fenoprop, fenoxaprop, fenoxaprop-p, fenoxaprop ethyl, fenoxaprop-p ethyl, fenoxasulfone, fentrazamide, fenuron, flamprop, flamprop-M-isopropyl, flamprop-M-methyl, flazasulfuron, florasuram, fluazifop, fluazifop-p, fluazifop butyl, fluazifop-p-butyl, fluazolate, flucarbazone, flucarbazone sodium, flucetosulfuron, fluchloralin, flufenacet, thiafluamide, flufenpyr, flufenpyr ethyl, flumetralin, flumetsulam, flumiclorac, flumiclorac-pentyl, flumioxazin, flumipropyn, fluometuron, fluorodifen, fluoroglycofen, fluoroglycofen-ethyl, flupoxam, flupropacil, flupropanate, flupyrsulfuron, flupyrsulfuron-methyl-sodium, flurenol, flurenol-butyl, fluridone, fluorochloridone, fluoroxypyr, fluoroxypyr-meptyl, flurprimidol, flurtamone, fluthiacet, fluthiacet-methyl, fluthiamide, fomesafen, foramsulfuron, forchlorfenuron, fosamine, furyloxyfen, gibberellic acid, glufosinate, glufosinate-ammonium, glufosinate-p, glufosinate-p-ammonium, glufosinate-p-sodium, glyphosate, glyphosate-isopropylammonium, H-9201, i.e., 0-(2, 4-dimethyl-6-nitrophenyl)-0-ethyl-isopropyl phosphoramide thioatethioate, halosafen, halosulfuron, halosulfuron-methyl, haloxyfop, haloxyfop-P, haloxyfop-ethoxyethyl, haloxyfop-P-ethoxyethyl, haloxyfop-methyl, haloxyfop-P-methyl, hexazinone, HW-02, i.e., (2,4-dichlorophenoxy)1-(dimethoxyphosphoryl)-ethyl acetate), imazamethabenz, imazamethabenz-methyl, imazamox, imazamox-ammonium, imazapic, imazapyr, imazapyr-isoprpyl ammonium, imazaquin, imazaquin-ammonium, imazethapyr, imazethapyr-ammonium, imazosulfuron, inabenfide, indanofan, indaziflam, indoleacetic acid (IAA), 4-indol-3-ylbutyric acid (IBA), iodosulfuron, iodosulfuron-methyl-sodium, ioxynil, indaziflam, isocarbamid, isopropalin, isoproturon, isouron, isoxaben, isoxachlortole, isoxaflutole, isoxapyrifop, KUH-043, i.e., 3-({[5-(difluoromethyl)-1-methyl-3- (trifluoromethyl)-1H-pyrazol-4-yl]methyl}sulfonil)-5,5-dimethyl-4,5-dihydro-1,2-oxazole, karbutilate, ketospiradox, lactofen, lenacil, linuron, maleic hydrazide, MCPA, MCPB, MCPB-methyl, -ethyl and -sodium, mecoprop, mecoprop-sodium, mecoprop-butotyl, mecoprop-P-butotyl, mecoprop-P-dimethylammonium, mecoprop-P-2-ethylhexyl, mecoprop-P-potassium, mefenacet, mefluidide, mepiquat-chloride, mesosulfuron, mesosulfuron-methyl, mesotrione, methabenzthiazuron, metam, metamifop, metamitron, metazachlor, metazasulfuron, methazole, methiopyrsulfuron, methiozolin, methoxyphenone, methyldymron, 1-methylcyclopropen, methyl isothiocyanate, metobenzuron, metobromuron, metolachlor, S-metolachlor, metosulam, metoxuron, metribuzin, metsulfuron, metsulfuron-methyl, molinate, monalide, monocarbamide, monocarbamide-dihydrogen sulfate, monolinuron, monosulfuron, monosulfuron ester, monuron, MT 128, i.e., 6-chloro-N-[(2E)-3-chloroprop-2-en-1-yl]-5-methyl-N-phenyl pyridazine-3-amine, MT-5950, i.e., N-[3-chloro-4-(1-methylethyl)-phenyl]-2-methyl pentanamide, NGGC-011, naproanilide, napropamide, naptalam, NC-310, i.e., 4-(2,4-dichlorobenzoyl)-1-methyl-5-benzyloxy pyrazole, neburon, nicosulfuron, nipyraclofen, nitralin, nitrofen, nitrophenolate-sodium (isomer mixture), nitrofluorfen, nonanoic acid, norflurazon, orbencarb, orthosulfamuron, oryzalin, oxadiargyl, oxadiazon, oxasulfuron, oxaziclomefone, oxyfluorfen, paclobutrazole, paraquat, paraquat-dichloride, pelargonic acid (nonanoic acid), pendimethalin, pendralin, penoxsulam, pentanochlor, pentoxazone, perfluidone, pethoxamid, phenisopham, phenmedipham, phenmedipham-ethyl, picloram, picolinafen, pinoxaden, piperophos, pirifenop, pirifenop-butyl, pretilachlor, primisulfuron, primisulfuron-methyl, probenazole, profluazole, procyazine, prodiamine, prifluraline, profoxydim, prohexadione, prohexadione-calcium, prohydrojasmone, prometon, prometryn, propachlor, propanil, propaquizafop, propazine, propham, propisochlor, propoxycarbazone, propoxycarbazone sodium, propyrisulfuron, propyzamide, prosulfalin, prosulfocarb, prosulfuron, prynachlor, pyraclonil, pyraflufen, pyraflufen-ethyl, pyrasulfotole, pyrazolynate (pyrazolate), pyrazosulfuron, pyrazosulfuron ethyl, pyrazoxyfen, pyribambenz, pyribambenz isopropyl, pyribenzoxim, pyributicarb, pyridafol, pyridate, pyriftalid, pyriminobac, pyriminobac methyl, pyrimisulfan, pyrithiobac, pyrithiobac-sodium, pyroxasulfone, pyroxsulam, benzyl aminopurine, quinclorac, quinmerac, quinoclamine, quizalofop, quizalofop-ethyl, quizalofop-P, quizalofop-P-ethyl, quizalofop-P-tefuryl, rimsulfuron, saflufenacil, secbumeton, sethoxydim, siduron, simazine, simetryn, SN-106279, i.e., methyl-(2R)-2({7-[2-chloro-4-(trifluoromethyl)phenoxy]-2-naphthyl)oxy)propanoate; sulcotrione, sulfallate (CDEC), sulfentrazone, sulfometuron, sulfometuron methyl, sulfosate (glyphosate-trimesium), sulfosulfuron, SYN-523, SYP-249, i.e., 1-ethoxy-3-methyl-1-oxobut-3-en-2-yl-5-[2-chloro 4-(trifluoromethyl)phenoxy]-2-nitro benzoate, SYP-300, i.e., 1-[7-fluoro-3-oxo-4-(prop-2-in-1-yl)-3,4-dihydro-2H-1,4-benzoxazine-6-yl]-3-propyl-2-thioxo imidazolidine-4,5-dione, tebutam, tebuthiuron, tecnazene, tefuryltrione, tembotrione, tepraloxydim, terbacil, terbucarb, terbuchlor, terbumeton, terbuthylazine, terbutryne, thenylchlor, thiafluamide, thiazafluoron, thiazopyr, thidiazimin, thidiazuron, thiencarbazone, thiencarbazone methyl, thiobencarb, tiocarbazil, topramezone, tralkoxydim, tri-allate, triasulfuron, triaziflam, triazofenamide, tribenuron, tribenuron methyl, trichloroacetic acid (TCA), triclopyr, tridiphane, trietazine, trifloxysulfuron, trifloxysulfuron-sodium, trifluralin, triflusulfuron, triflusulfuron-methyl, trimeturon, trinexapac, trinexapac-ethyl, tritosulfuron, tsitodef, uniconazole, uniconazole-P, vernolate, ZJ-0862, i.e., 3,4-dichloro-N-{2-[(4,6-dimethoxypyrimidine-2-yl)oxy]benzyl}aniline, and the following compounds: 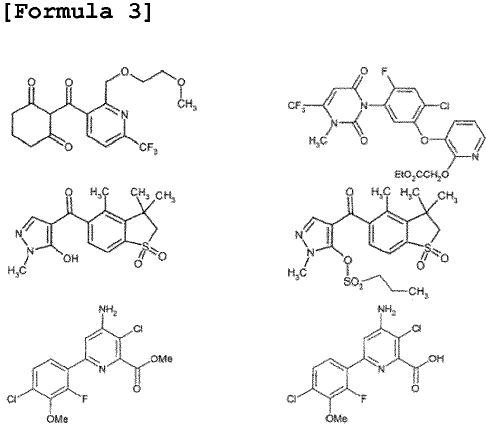 MBH-024 flowable (Herbicide/Growth Regulator Test Results, Japan Association for Advancement of Phyto-Regulators).
Particularly preferable secondary ingredients include:
oryzalin, pendimethalin, cafenstrole, lenacil, pyributicarb, imazaquin ammonium, quinoclamin, propyzamide, prodiamine, napropamide, DCBN, halosulfuron methyl, cyanazine, flupoxam, ethoxysulfuron, S-metolachlor, indaziflam, isoxaben, florasuram, triaziflam, butamifos, dithiopyr, indanofan, bethrodine, alachlor, oxadiargyl, oxaziclomefone, imazosulfuron, asulam, chlorimuron ethyl, mecoprop P potassium salt, mecoprop, ethoxysulfuron, metsulfuron methyl, triclopyr, flazasulfuron, imazosulfuron, pyraflufen ethyl, carfentrazone ethyl, cyclosulfamuron, iodosulfuron methyl sodium salt, foramsulfuron, rimsulfuron, MDBA, MCPA isopropyl amine salt, benfuresate, flucetosulfuron, flufenacet, trifloxysulfuron sodium, metamifop, fluazifop, and flufenacet, but the present invention is not limited to these.

In a preferred embodiment of the present invention, a penetrant is additionally added to a crop protection composition. Suitable penetrants include, for example, substances enhancing the usefulness of the compound of formula (1) in a sprayed coating. Examples of such penetrants include mineral oil, vegetable oil, and the like. Suitable oils are all mineral oils and vegetable oils (modifiable as necessary) generally used in agrochemical compositions. Examples include sunflower oil, rapeseed oil, olive oil, castor oil, colza oil, maize seed oil, cottonseed oil, soybean oil, and esters thereof. Rapeseed oil, sunflower oil, and methyl esters and ethyl esters thereof are preferable, and rapeseed oil methyl esters are especially preferable.

The concentration of penetrant within the composition of the present invention can be modified within a broad range. In a prepared crop protection composition, the concentration will generally be from 1 to 95 mass%, preferably from 1 to 55 mass%, especially preferably from 15 to 40 mass%. In a ready-to-use composition (spray), the concentration will generally be from 0.1 g/L to 10 g/L, preferably from 0.5 g/L to 5 g/L.

The composition is applied using a conventional method as suits the form in which it is used.

Treatment of a plant or parts of a plant using the active compound according to the present invention may be performed using a conventional treatment method, such as direct application to lawn via spraying, scattering, or dispersing, or by spraying onto, pouring onto, or mixing with soil in which lawn grows.

If applied by spray treatment, the amount of penflufen is from 10 ppm to 1,000 ppm, preferably from 50 ppm to 800 ppm, especially preferably from 100 ppm to 400 ppm.

In one embodiment of the present invention, from 10 ppm to 1,000 ppm penflufen is applied to promote lawn growth.

In one embodiment of the present invention, from 50 ppm to 800 ppm penflufen is applied to promote lawn growth.

In one embodiment of the present invention, from 100 ppm to 400 ppm penflufen is applied to promote lawn growth.

There is no particular limitation upon when spray application is performed; preferable times are immediately following sodding, after the lawn has taken root, the period from immediately after lawn seed planting to 3.0 leaf cycles, the period from immediately after lawn seed planting to 2.0 leaf cycles, the period from immediately after lawn seed planting to 1.0 leaf, the period from immediately after lawn seed planting to 0.5 leaf cycles, and at 3.0 leaf cycles or later.

In one embodiment of the present invention, penflufen is applied within a period from immediately after lawn seed planting to 3.0 leaf cycles in order to promote lawn growth.

In one embodiment of the present invention, penflufen is applied within a period from immediately after lawn seed planting to 2.0 leaf cycles in order to promote lawn growth.

In an embodiment of the present invention, penflufen is applied within a period from immediately after lawn seed planting to 1.0 leaf cycles in order to promote lawn growth.

Examples of the present invention will be given in the form of the following biological examples, but the present invention is not limited to these biological examples.

### [Working Examples]

### Biological example 1:

### Test crop: Zoysia Matrella

Test method: *Z. Matrella* sod was laid in a roughly 15 cm checked pattern, and, approximately one month later, penflufen prepared to a predetermined concentration (Cerenturf Forte; aqueous suspension of 22.7 wt% penflufen and 77.3% surfactant and water, prepared according to a normal method) was sprayed thereon (2,000 L/ha). 170 days later, a hole cutter was used to cut out sod sections and interstitial sections, the sections were separated into roots, runners, and erect stems, and the weight of each was measured.

**[Table 1]**

| Interstitial section weight | | | | | | | | |
|---|---|---|---|---|---|---|---|---|
| | Treatment chemical | Roots weight (g) | vs. untreated | Runner weight (g) | Erect stem weight (g) | Roots / (roots + runners + erect stems) | Roots / runners | Erect stems / runners |
| Penflufen | 334 ppm | 1.13 | 127% | 3.70 | 4.57 | 12.02% | 31% | 124% |
| Untreated | | 0.89 | 100% | 3.31 | 4.03 | 10.81% | 27% | 122% |

**[Table 2]**

| Sod section weight | | | | | | | | |
|---|---|---|---|---|---|---|---|---|
| | Treatment chemical | Roots weight (g) | vs. untreated | Runner weight (g) | Erect stem weight (g) | Roots / (roots + runners + erect stems) | Roots / runners | Erect stems / runners |
| Penflufen | 334 ppm | 1.57 | 103% | 8.84 | 11.52 | 7.16% | 18% | 130% |
| Untreated | | 1.52 | 100% | 11.31 | 9.94 | 6.68% | 13% | 88% |

There was an increase in the amount of roots in both the interstitial section and the sod section in the penflufen-treated area compared to the untreated area. There was an especially marked increase in the interstitial section. The ratio of the root weight to the total weight of the roots, runners, and erect stems was also higher in the treated area than in the untreated area.

An embodiment of the present invention relates to the use of penflufen to promote lawn growth in sodded areas when lawn propagation is effected through vegetative propagation (sodding), i.e., through runner elongation (internode elongation).

### Biologic example 2

### Test crop: Agrostis stolonifera

Test method: Plastic cups (diameter 20 cm, depth 5 cm) were filled with a soil mixture (1:1 mixture of sand and mulch), the soil was treated (2,000 L/ha water) with penflufen prepared to a predetermined concentration (Cerenturf Forte) on the day *A*. *stolonifera* was planted and at 0.5 leaf cycles, after which germination state was examined. In order to ensure evenness of irrigation, an atomizer was used for a fixed number of pushes, and water amount was monitored so as to be uniform. A basal application was not used.

Examination method: Cuttings were taken at random from the test area, and the lengths of the above-ground and root portions were measured.

**[Table 3]**

| | Amount of agent | Foliar age at time of treatment | Days after treatment | Above-ground portion (mm) | Roots (mm) | Roots / above-ground portion % |
|---|---|---|---|---|---|---|
| Penflufen | 283 ppm | Day of planting | 20 days | 17.3 | 3.2 | 18.8% |
| Penflufen | 283 ppm | 0.5 leaf cycles | 5 days | 17.3 | 3.5 | 20.1% |
| Penflufen | 142ppm | Day of planting | 20 days | 15.9 | 3.3 | 20.8% |
| Penflufen | 142 ppm | 0.5 leaf cycles | 5 days | 18.8 | 3.9 | 20.6% |
| Thifluzamide | 525 ppm | Day of planting | 20 days | 15.3 | 1.4 | 9.1% |
| Thifluzamide | 525 ppm | 0.5 leaf cycles | 5 days | 16.9 | 2.0 | 12.0% |
| Untreated | | | | 18.2 | 2.4 | 13.1% |

For both registered weight (283 ppm) and half-weight penflufen treatment, root growth tended to be promoted over that of above-ground portions at planting day and 0.5 leaf cycle treatment.

An embodiment of the present invention relates to the use of penflufen to promote lawn root elongation while suppressing above-ground lawn elongation when lawn propagation is effected by seeding.

## Claims

1. Use of a compound (chemical name: N-[2-(1,3-dimethylbutyl)phenyl]-5-fluoro-1,3-dimethyl-1H-pyrazol-4-carboxamide; generic name: penflufen) as represented in the following formula (I), or an agriculturally acceptable salt thereof, to promote lawn growth through lawn root elongation and suppression of above-ground lawn elongation.

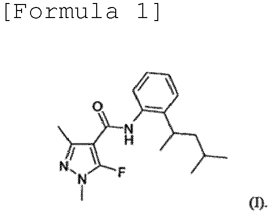

2. The use according to claim 1, wherein lawn propagation is vegetative propagation (sodding), and lawn growth in sodded areas is promoted.

3. The use according to any one of claims 1 to 2, wherein the compound of formula (I) is used in combination with one or more active ingredients selected from the group consisting of germicides, bactericides, acaricides, nematicides, insecticides, microbicides, fertilizers, attractants, sterilants, synergists, safeners, information chemical substances, and plant growth adjusters.

4. The use according to any of claims 1 to 3, wherein the amount of the compound of formula (I) applied is from 100 ppm to 400 ppm.

## Patentansprüche

1. Verwendung einer Verbindung (chemischer Name: N-[2-(1,3-Dimethylbutyl)phenyl]-5-fluor-1,3-dimethyl-1H-pyrazol-4-carboxamid; generischer Name: Penflufen) gemäß der folgenden Formel (I), oder eines landwirtschaftlich unbedenklichen Salzes davon, zur Förderung des Rasenwachstums über das Längenwachstum der Rasenwurzeln und Unterdrückung des oberirdischen Längenwachstums des Rasens.

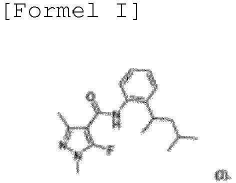

2. Verwendung nach Anspruch 1, wobei es sich bei der Rasenvermehrung um vegetative Vermehrung (Sodenlegung) handelt und das Rasenwachstum auf den berasten Flächen gefördert wird.

3. Verwendung nach einem der Ansprüche 1 bis 2, wobei die Verbindung der Formel (I) in Kombination mit einem oder mehreren Wirkstoffen, ausgewählt aus der Gruppe bestehend aus keimtötenden Mitteln, Bakteriziden, Akariziden, Nematiziden, Insektiziden, Mikrobiziden, Düngemitteln, Attractants, Sterilantien, Synergisten, Safener und Pflanzenwachstumsregulatoren, verwendet wird.

4. Verwendung nach einem der Ansprüche 1 bis 3, wobei die eingesetzte Menge der Verbindung der Formel (I) 100 ppm bis 400 ppm beträgt.

## Revendications

1. Utilisation d'un composé (nom chimique : N-[2-(1,3-diméthylbutyl)phényl]-5-fluoro-1,3-diméthyl-1H-pyrazol-4-carboxamide ; nom générique : penflufène) tel que représenté dans la formule (I) suivante, ou un sel acceptable sur le plan agricole de celui-ci, afin de favoriser la croissance de gazon par l'intermédiaire de l'élongation des racines du gazon et la suppression de l'élongation des parties aériennes du gazon

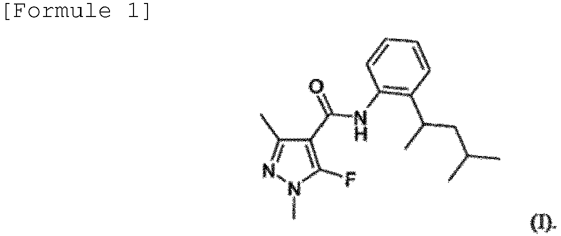

2. Utilisation selon la revendication 1, dans laquelle la propagation du gazon est une propagation végétative (gazonnement), et la croissance du gazon dans des zones engazonnées est favorisée.

3. Utilisation selon l'une quelconque des revendications 1 à 2, dans laquelle le composé de formule (I) est utilisé en combinaison avec un ou plusieurs ingrédients actifs choisis dans le groupe constitué par les germicides, les bactéricides, les acaricides, les nématicides, les insecticides, les microbicides, les fertilisants, les attractifs, les stérilisants, les agents synergiques, les phytoprotecteurs et les substances de croissance.

4. Utilisation selon l'une quelconque des revendications 1 à 3, dans laquelle la quantité du composé de formule (I) appliquée va de 100 ppm à 400 ppm.
